# EUROPÄISCHE PATENTSCHRIFT

(11) **EP 1 594 500 B1**
(45) Veröffentlichungstag und Bekanntmachung des Hinweises auf die Patenterteilung: **29.09.2010**
(21) Anmeldenummer: 04705765.8
(22) Anmeldetag: 28.01.2004
(51) Int. Cl.: A61K 31/496, A61K 9/14, A61P 31/04

(54) **BEHANDLUNG BAKTERIELLER ERKRANKUNGEN DER ATMUNGSORGANE DURCH LOKALE APPLIKATION VON FLUORCHINOLONEN**
TREATMENT OF BACTERIAL DISEASES OF THE RESPIRATORY ORGANS BY LOCALLY APPLYING FLUOROQUINOLONES
TRAITEMENT DE MALADIES BACTERIENNES DES ORGANES RESPIRATOIRES PAR APPLICATION LOCALE DE FLUOROQUINOLONES

(30) Priorität: 10.02.2003 DE 10305319; 10.02.2003 DE 10305318
(43) Veröffentlichungstag der Anmeldung: 16.11.2005
(73) Patentinhaber: Bayer Schering Pharma Aktiengesellschaft, 13353 Berlin (DE)
(72) Erfinder: ENDERMANN, Rainer, 42113 Wuppertal (DE); LABISCHINSKI, Harald, D 14199 Berlin (DE); LADEL, Christoph, I 10010 Loranzé (To) (IT); PETERSEN, Uwe, 51375 Leverkusen (DE); NEWTON, Ben, Buckinghamshire HP6 6BX (GB)
(86) Internationale Anmeldenummer: PCT/EP2004/000710
(87) Internationale Veröffentlichungsnummer: WO 2004/069253

(56) Entgegenhaltungen:
- EP-A- 0 209 000
- EP-A- 0 504 112
- WO-A-01/32144
- WO-A-03/090715

## Beschreibung

Die vorliegende Erfindung betrifft die Verwendung Ciprofloxacin-Betain enthaltender Darreichungsformen, die eine lokale Behandlung von Erkrankungen der Atmungsorgane, insbesondere bakteriell verursachter Lungenkrankheiten, ermöglichen.

Trotz enormer Fortschritte auf dem Gebiet der Bekämpfung bakterieller Infektionskrankheiten durch die Einführung verschiedener Antibiotikaklassen in den letzten 70 Jahren stellen schwere Lungeninfektionen auch heute noch ein wichtiges Problem dar, insbesondere hinsichtlich Krankheitsbildern wie der cystischen Fibrose, der Bronchiektasis und zunehmend auch der chronisch obstruktiven Atemwegserkrankungen (COPD), die mit nur schwierig bis gar nicht zu behandelnden Infektionen einhergehen. Häufig werden für die Behandlung derartiger Erkrankungen wegen Ihres relevanten antibakteriellen Wirkspektrums und ihrer bakteriziden Wirkung Wirkstoffe aus der Klasse der Fluorchinolone, insbesondere auch Moxifloxacin und Ciprofloxacin eingesetzt.

Moxifloxacin-Hydrochlorid (I) ist ein antibakterieller Wirkstoff aus der

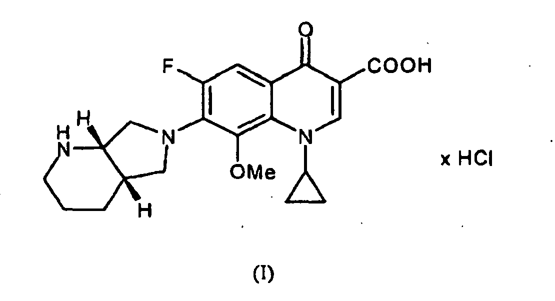

Klasse der Chinoloncarbonsäurederivate zur Behandlung und Verhinderung von Otitis, Pharyngitis, Pneumonie, Peritonitis, Pyelonephritis, Cystitis, Endocarditis, Systeminfektionen, akuter und chronischer Bronchitis, septischen Infektionen, Erkrankungen der oberen Luftwege, diffuser Panbronchiolitis, pulmonärem Emphysem, Dysenterie, Enteritis, Leberabzessen, Urethritis, Prostatitis, Epididymitis, gastrointestinalen Infektionen, Knochen- und Gelenkinfektionen, zystischer Fibrose, Hautinfektionen, postoperativen Wundinfektionen, Abszessen, Phlegmonen, Wundinfektionen, infizierten Verbrennungen, Brandwunden, Infektionen im Mundbereich, Infektionen nach Zahnoperationen, Ostcomyelitis, septischer Arthritis, Cholecystitis, Peritonitis mit Appendicitis, Cholangitis, intraabdominalen Abszessen, Pankreatitis, Sinusitis, Mastoiditis, Mastitis, Tonsillitis, Typhus, Meningitis, Infektionen des Nervensystems, Salpingitis, Endometritis, Genital-Infektionen, Pelveoperitonitis und Augeninfektionen (EP 350 733 B1, US 4 990 517, 5 607 942 und WO 01/ 45679). Die wichtigsten Indikationen für Moxifloxacin sind Erkrankungen des Respirationstraktes, insbesondere der Lunge. ,

Als Darreichungsformen von Moxifloxacin nennt die EP-B 350 733 Tabletten, Dragees, Kapseln, Pillen, Granulate, Suppositorien, Lösungen, Suspensionen und Emulsionen, Pasten, Salben, Gele, Cremes, Lotions, Puder, und Sprays. Auf dem Markt sind unseres Wissens nur Tabletten und (für die intravenöse Verabreichung) Lösungen.

In der Praxis wird Moxifloxacin bis heute bei allen Erkrankungen, für deren Bekämpfung es in Frage kommt (einscliließlich denen der Lunge), ausschließlich systemisch angewandt. Der Grund dafür liegt in der hohen oralen Bioverfügbarkeit und in der guten Verteilung des Wirkstoffs. Die Wirkstoffkonzentration in Serum und Lunge von Ratten nach lokaler (intratrachealer) Applikation von Moxifloxacin-Hydrochlorid steigt zwar, verglichen mit der systemischen (oralen) Applikation der gleichen Menge Moxifloxacin-Hydrochlorid, stärker an, aber diese Konzentration fällt auch relativ rasch (etwa innerhalb einer Stunde) auf das Niveau der oral erzielten Konzentration ab, so dass auch in Versuchen an der Ratte eine intratracheale lokale Verabreichung keine Vorteile gegenüber einer oralen Applikation erzielt wird.

Ciprofloxacin- und Enrofloxacin-Hydrochlorid (II) sind seit ca. 20 Jahren bekannte antibakterielle Chinoloncarbonsäurederivate (EP-B 49 355, US-PS 4 670 444), die äußerst erfolgreich sowohl zur Prophylaxe als auch zur Behandlung von systemischen und lokalen bakteriellen Infektionen, insbesondere der Harnwege eingesetzt werden können. Ciprofloxacin ist u.a. auch gegen Milzbranderreger wirksam.

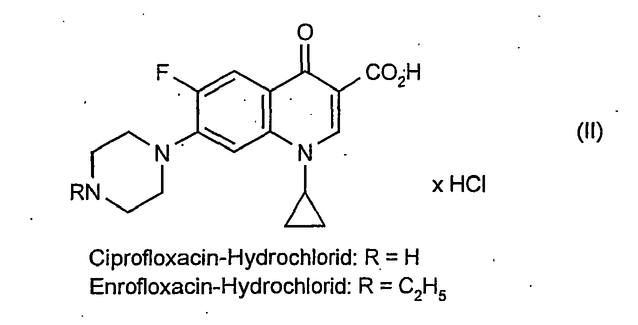

Als Darreichungsformen von Ciprofloxacin/Enrofloxacin nennt die EP-B 49 355 Tabletten, Dragees, Kapseln, Pillen, Granulate, Suppositorien, Lösungen, Suspensionen und Emulsionen, Pasten, Salben, Gele, Cremes, Lotionen, Puder und Sprays. Auf dem Markt sind von Ciprofloxacin derzeit Tabletten, Suspensionen, Augen- und Ohrentropfen und zur intravenösen Infusion geeignete Lösungen.

WO01/32144 A beschreibt inhalierbare Trockenpulverzubereitung enthaltend Di- oder Tripeptide, wobei Ciprofloxacin als eine Beispielsubstanz unter mehreren Fluorchinolonen und weiteren Wirkstoffen anderer Wirkstoffklassen genannt wird. Die Trockenpulverzubereitungen zeigen eine vorteilhafte Stabilität und Dispersierbaikeit. Eine Beispielformulierung mit Ciprofloxaein-Hydrochlorid wird offenbart.

EP 0504112 A offenbart Suspendierhilfsmittel für Aeresolformulierungen mit Wirkstoffen in "alternativen" Treibmitteln. Auch hier wird Ciprofloxacin als eine Beispielsubstanz unter mehreren Antibiotika und weiteren Wirkstoffen anderer Wirkstoffklassen in Aufzählung genannt.

WO03/090715 A beschreibt partikuläre Trockenpulverformulierungen zur Inhalation mit speziellen Partikeleigenschaften wie Partikeloberflächenenergie, Größe und Größenverteilung, Kristallinitätsgrad, morphologische Einheitlichkeit etc. Ciprofloxacin wird wieder als eine Beispielsubstanz unter mehreren Fluorchinolonen und weiteren Wirkstoffen anderer Wirkstoffklassen genannt.

Überraschenderweise wurde gefunden, dass die Bekämpfung von Erkrankungen der Atmungsorgane, insbesondere bakteriell verursachter Lungenkrankheiten, dann überaus erfolgreich verläuft, wenn man Ciprofloxacin als festes Betain lokal appliziert. Die Wirkstoffkonzentrationen in der Lunge können über längere Zeit auf einem Niveau gehalten werden, wie es aus ärztticher Sicht zur optimalen Behandlung wünschenswert ist. Neben dem hohen und lang anhaltenden Wirkstoffspiegel am Ort der Infektion lässt sich gleichzeitig eine vergleichsweise niedrige systemische Konzentration des Wirkstoffs eaielen, so dass auf diesem Wege Nebenwirkungen der Medikation und die gefürchtete Resistenzentwicklung durch systemischen Selektionsdruck zumindest drastisch reduziert oder gar ganz vermieden werden.

Gegenstand der Erfindung ist also ein Verfahren zur Herstellung eines Arzneimittels zur Bekämpfung von bakterieller Erkrankungen der Lungen verursacht durch *P. aeruginosa* in Menschen und Tieren durch lokale Applikation einer antibakteriell wirksamen Menge von festem Betain der Formel (III) 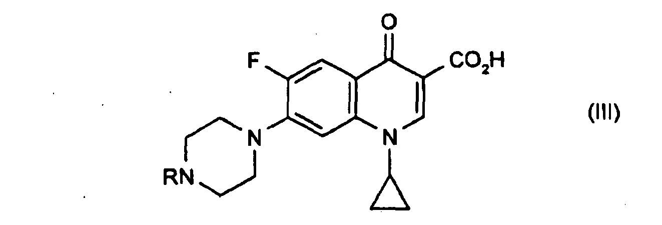 R=H
und die Verwendung dieser Verbindungen zur Herstellung von Arzneimitteln zur lokalen Bekämpfung von bakteriellen Erkrankungen der Lungen verursacht durch P. *aeruginosa* in Menschen und Tieren, wobei die Verbindungen in fester Form appliziert werden.

Erfindungsgemäß handelt es sich bei der oder den Erkrankungen der Atmungsorgane um bakteriell bedingte Erkrankungen der Atemwege oder der Lunge, insbesondere um bakteriell verursachte Lungenkrankheiten.

Der Begriff "Atmungsorgane" (engl.: respiratory system) bezeichnet im Sinne der Erfindung Nase, Mundhöhle und Schlund sowie Kehlkopf, Luftröhre und Lungen einschließlich der Atemwege sowie der Nasennebenhöhlen und Stirnhöhle, wobei unter "Atemwege" (engl.: respiratory tract) Nasenhöhle, Mundhöhle, Rachen, Kehlkopf, Luftröhre und Bronchien verstanden wird.

Unter "lokaler Applikation" bzw. "lokaler Bekämpfung" im Zusammenhang mit Lungenkrankheiten, wird im Sinne der Erfindung - im Gegensatz zur oralen Applikation von zur Aufnahme über den Gastrointestinaltrakt vorgesehenen Darreichungsformen und im Gegensatz zur intravenösen Applikation - die inhalative Applikation des Wirkstoffs in inhalierbarer Darreichungsform verstanden. Bei den erfindungsgemäß zu verwendenden pulverförmige Zubereitungen handelt es sich um Zubereitungen, die zerstäubt und dann inhaliert werden.

Der Begriff "inhalativ" bzw. "inhalative Applikation" bezeichnet dabei die Einbringung in die Atmungsorgane, insbesondere in und/oder über die Atemwege, vorzugsweise in und/oder über Nasenhöhle und Mundhöhle.

Der Begriff "intratracheal" bzw. "intratracheale Applikation" bezeichnet im Sinne der Erfindung die nicht-inhalative Einbringung in die Luftröhre, insbesondere zur pulmonalen Krankheitsbekämpfung in Versuchstieren wie Ratten als Modell für die inhalative Applikation.

Im Zusammenhang mit der Erfindung offenbart sind auch Vorrichtungen, die Betain (III) enthaltende Zubereitungen beinhalten und zur inhalativen Applikation derselben in fester Form geeignet sind, also Zerstäuber, die Betain (III) enthaltende Zubereitungen inhalativ in fester Form applizieren können (Pulverinhalatoren).

Feste Zubereitung zur Dry-powder-Inhalation werden im Allgemeinen einen möglichst hohen Wirkstoffanteil (d.h. Betain (III)) enthalten. Sie besitzen in der Regel einen Wirkstoffanteil von mindestens 60, vorzugsweise mindestens 70, insbesondere mindestens 80 und höchst bevorzugt mindestens 90 Gew.-%, bezogen auf die gebrauchsfertige Zubereitung. Sofern keine Hilfsmittel notwendig sind, können sie auch allein aus Wirkstoff bestehen. Aus praktischen Gründen handelt es sich bei den erfindungsgemäßen Zubereitungen aber oft um Arzneimittel, die neben dem Wirkstoff einen oder mehrere pharmakologisch unbedenkliche Hilfsstoffe enthalten. Eine Übersicht über verschiedene geeignete Zubereitungen und entsprechende Verabreichungshilfen findet man z.B. in R. Stangl, "An Overview of Innovative Inhalation Devices", European Pharmaceutical Review, Seiten 50-55, (2002) und in der dort zitierten Literatur. Zu den pharmakologisch unbedenklichen Hilfsstoffen zählen u.a. Bindemittel (z.B. Maisstärke, Gelatine), Stabilisatoren (z.B. Antioxidantien wie Ascorbinsäure), Trägerstoffe (z.B. mikrokristalline Cellulose, Lactose, Sucrose, Calciumphosphat, Maisstärke), Gleitmittel (z.B. Talk, Stearinsäure, Magnesium-, Calcium- oder Zinkstearat), Aromastoffe und/ oder Duftstoffe. Die Herstellung geeigneter Zubereitungen durch Auswahl geeigneter Hilfsstoffe nach Art und Menge ist unproblematisch.

Die Herstellung der erfindungsgemäßen Zubereitungen kann - wie bei der Herstellung inhalierbarer frei fließender pulverförmiger Arzneimittel üblich - durch Mikronisierung des Wirkstoffs oder durch Sprühtrocknung entsprechender Lösungen oder Suspensionen erfolgen.

Die festen Zubereitungen weisen im Allgemeinen als Volumenmittel (mit Hilfe eines Laserbeugungsgeräts) bestimmte Teilchendurchmesser von 0,2 bis 15 µm, vorzugsweise von 1 bis 5 µm, auf. Der als Vofumenmittel bestimmte Durchmesser ist der Wert, unterhalb und oberhalb dessen jeweils 50 % des Volumens der Teilchen liegen.

In einer bevorzugten Ausführungsform weisen die festen Zubereitungen als 50-%-Volumenmittel bestimmte Teilchendurchmesser von 2 bis 5 µm und als 90%-Volumenanteil bestimmte Teilchendurchmesser von 6 bis 10 µm auf. In einer weiteren bevorzugten Ausführungsform enthalten die festen Zubereitungen Wirkstoff, insbesondere Ciprofloxacin Betain, mit einem als 50%-Volumenmittel bestimmten Teilchendurchmesser von 2 bis 5 µm und als 90-%-Volumenanteil bestimmten Teilchendurchmesser von 6 bis 10 µm. Der als 50-%- beziehungsweise 90%-Volumenanteil (mit Hilfe eines L.aserbeugungsgeräts) bestimmte Durchmesser ist der Wert, unterhalb dessen 50 % beziehungsweise 90 % des Volumens der Teilchen liegen. So liegt beispielsweise in einer festen Zubereitungen mit einem als 50-%-Volumenmittel bestimmten Teilchendurchmesser von 2 µm und als 90-%-Volumenanteil bestimmten Teilchendurchmesser von 6 µm (50 % < 2 µm; 90 % < 6 µm) der Teilchendurchmesser von 50 % des Volumens der Teilchen unterhalb 2 µm und von 90 % des Volumens der Teilchen unterhalb 6 µm.

Im Allgemeinen hat es sich als vorteilhaft erwiesen, bei inhalativer Applikation Mengen von etwa 0,1 bis 20, vorzugsweise etwa 0,5 bis 7,5 mg/kg Körpergewicht zur Erzielung wirksamer Ergebnisse zu verabreichen.

Trotzdem kann es gegebenenfalls erforderlich sein, von den genannten Mengen abzuweichen, und zwar in Abhängigkeit von Körpergewicht, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

### Beispiele

### Herstellung von Ciprofloxacin-Embonat (nicht erfindungsgemäß)

### a) Ciprofloxacin-Embonat (IV a; R = H)

33,1 g (0,1 mol) Ciprofloxacin-Betain und 38,8 g (0,1 mol) Embonsäure werden in 500 ml Glykolmonomethylether 1 Stunde unter Rückfluss erhitzt. Nach dem Abkühlen wird der Niederschlag abgesaugt, gut mit Ethanol gewaschen und im Hochvakuum bei 120°C getrocknet.

### b) Ciprofloxacin-Hemi-Embonat (IV b; R = H)

66,2 g (0,2 mol) Ciprofloxacin-Betain und 38,8 g (0,1 mol) Embonsäure werden in 500 ml Glykolmonomethylether 1 Stunde unter Rückfluss erhitzt. Nach dem Abkühlen wird der Niederschlag abgesaugt, gut mit Ethanol gewaschen und im Hochvakuum bei 120°C getrocknet.

### Bestimmung der Wirkstoffkonzentration in Lungen von Ratten

Weibliche Wistar-Ratten (80 bis 100 g) wurden behandelt mit
A1 - 7,5 mg/kg Ciprofloxacin-Betain (Laborware) intratracheal als Suspension;
A2 - 7,5 mg/kg Ciprofloxacin-Betain (mikronisiert: 50 % < 3 µm; 90 % < 7 µm) intratracheal als Suspension;
B - 7,5 mg/kg Ciprofloxacin-Hydochlorid intratracheal als Lösung;
C - 7,5 mg/kg Ciprofloxacin-Hydrochlorid intravenös als Lösung.

Je drei Tiere aus den Dosisgruppen wurden nach 0,25; 0,5; 1; 3 und 5 Stunden getötet und die Lungen entnommen. Die Lungen wurden mit Hilfe eines Plotters der Fa. Braun homogenisiert. Durch Bioassay wurde der Wirkstoffgehalt in den Lungenhomogenisaten bestimmt.

### Pharmakokinetikparameter der Lungen

| **Dosisgruppe** | **AUC [mg*h/l]** | **Cₘₐₓ [mg/l]** | **t_{1/2} [h]** |
|---|---|---|---|
| A1 | 124 | 49,1 | 1,96 |
| A2 | 304 | 76 | 13,47 |
| B | 3,20 | 6,65 | 0,847 |
| C | 0,497 | 0,610 | 0,758 |

**Lungenkonzentrationen in µg/ml; Mittelwerte von 3 Tieren**

| **Dosisgruppe** | **0,25 Std.** | **0,5 Std.** | **1 Std.** | **3 Std.** | **5 Std.** |
|---|---|---|---|---|---|
| A1 | 49,1 | 44,3 | 40,1 | 12,6 | 7,21 |
| A2 | 76 | 76 | 76 | 54 | 54 |
| B | 6,65 | 1,67 | 0,63 | 0,10 | 0 |
| C | 0,61 | 0,35 | 0,14 | 0 | 0 |

AUC, Cₘₐₓ und t_{1/2} sind wichtige pharmakokinetische Parameter für die Beschreibung von Pharmakokinetik/Pharmakodynamik-Interaktionen; vgl. z.B. W.A. Craig, "Pharmacokinetic/pharmacodynamic parameters: rationale for antibacterial dosing of mice and men", Clin. Infect. Dis. 26, 1-12 (1998).

Die intratracheale Applikation von Ciprofloxacin-Betain (Laborware) ergibt, verglichen mit der intratrachealen Gabe von Ciprofloxacin-Hydrochlorid, eine 40-fach größere AUC und eine 8-fach höhere Cₘₐₓ. Dieser kinetische Vorteil wird noch deutlicher, wenn man mit der intravenösen Applikation von Ciprofloxacin-Hydrochlorid vergleicht (AUC: Faktor 250, Cₘₐₓ: Faktor 80). Die intratracheale Applikation von mikronisiertem Ciprofloxacin-Betain mit einem Teilchendurchmesser (50 % < 3 µm; 90 % < 7 µm) führt zu einer weiteren Verbesserung des pharmakokinetischen Profils (AUC: Faktor 612, Cₘₐₓ: Faktor 125, verglichen mit intravenöser Applikation von Ciprofloxacin-Hydrochlorid).

### Wirksamkeit im Lungeninfektionsmodell mit P. aeruginosa

Weibliche Wistar-Ratten (80 bis 100 g) wurden intratracheal mit P. aeruginosa DSM 12055 infiziert und 1 und 4 Stunden nach der Infektion mit Ciprofloxacin-Betain (Laborware) intratracheal (i.t.) bzw. mit Ciprofloxacin-Hydrochlorid intratracheal bzw. intravenös (i.v.) behandelt. Es wurden verschiedene Dosisgruppen mit jeweils 5 Ratten eingesetzt. 24 Stunden nach der Infektion wurden die Tiere getötet, die Lungen entnommen und diese mit Hilfe eines Potters der Fa. Braun homogenisiert. Zur Bestimmung der Keimzahl in den Lungen wurden die Homogenate plattiert.

Die folgende Tabelle zeigt die **Keimzahlreduktion in den Lungen** (log-Einheiten) nach 24 Std., bezogen auf die unbehandelte Infektionskontrolle (jeweils Mittelwerte der 5 Tiere):

| **Dosisgruppe** | **Keimzahlreduktion (log-Einheiten)** |
|---|---|
| A - 0,8 mg/kg Ciprofloxacin-Betain i.t. | -4,0 |
| B - 2,5 mg/kg Ciprofloxacin-Betain i.t. | -5,8 |
| C - 7,5 mg/kg Ciprofloxacin-Betain i.t. | -10 |
| D - 0,8 mg/kg Ciprofloxacin-Hydrochlorid i.t. | -1,8 |
| E - 2,5 mg/kg Ciprofloxacin-Hydrochlorid i.t. | -1,1 |
| F - 7,5 mg/kg Ciprofloxacin-Hydrochlorid i.t. | -2,8 |
| G - 2,5 mg/kg Ciprofloxacin-Hydrochlorid i.v.. | -2,8 |
| H - 7,5 mg/kg Ciprofloxacin-Hydrochlorid i.v. | -3,5 |
| I - 22,5 mg/kg Ciprofloxacin-Hydrochlorid i.v. | -5,4 |
| J - 67,5 mg/kg Ciprofloxacin-Hydrochlorid i.v. | -8,8 |

Die intratracheale Anwendung von Ciprofloxacin-Betain führt in allen drei Dosisgruppen zu einer Keimzahlreduktion von 4 bis 10 log-Einheiten in den Lungen, während gleiche Dosierungen von Ciprofloxacin-Hydrochlorid, intratracheal appliziert, eine weitaus geringere Reduktion der Keimzahl bewirken (1,1 bis 2,8 log-Einheiten). Die intravenöse Gabe von Ciprofloxacin-Hydrochlorid, der gegenwärtige Stand der Technik, führt zwar auch zu einer deutlichen Keimzahlreduktion in den Lungen (2,8 bis 8,8 log-Einheiten), allerdings erst bei 10-fach höheren Dosierungen. Die erfindungsgemäße Behandlung führt also zu einer stark verringerten systemischen Belastung.

In einem weiteren Test mit P.aeruginosa DSM 12055 wurde eine einmalige intratracheale Behandlung (1 Stunde nach der Infektion) mit mikronisiertem Ciprofloxacin-Betain als "dry powder" durchgeführt. Eine Dosis von 10 mg/kg wurde mit Hilfe eines DP-3 Dry Powder Insufflators (PENN-CENTURY, INC.) appliziert. Aus Kontrolluntersuchungen ist bekannt, dass etwa 5 bis 20 % der applizierten Dosis die Lungen erreichen. In diesem Experiment wurde die Keimzahl in den Lungen im Vergleich zu einer unbehandelten Kontrollgruppe um mehr als 6 log-Einheiten reduziert.

## Patentansprüche

1. Verfahren zur Herstellung eines Arzneimittels zur Bekämpfung bakterieller Erkrankungen der Lungen verursacht durch *P. aeruginosa* in Menschen und Tieren durch lokale Applikation einer antibakteriell wirksamen Menge von festem Betain der Formel (III)

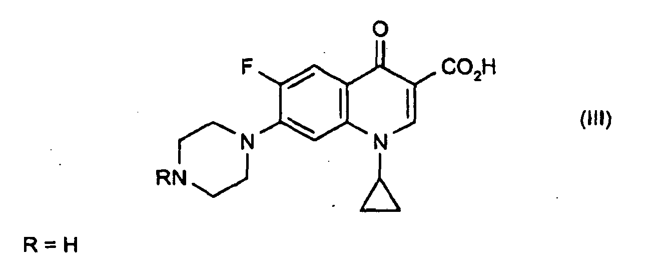

2. Verwendung der in Anspruch 1 definierten Verbindung zur Herstellung von Arzneimitteln zur lokalen Bekämpfung bakterieller Erkrankungen der Lungen verursacht durch *P. aeruginosa* in Menschen und Tieren, wobei diese Verbindung in fester Form appliziert werden.

## Claims

1. Method for producing a medicament controlling bacterial diseases of the lungs caused by *P. aeruginosa* in humans and animals by local administration of an antibacterially effective amount of solid betaine of the formula (III) 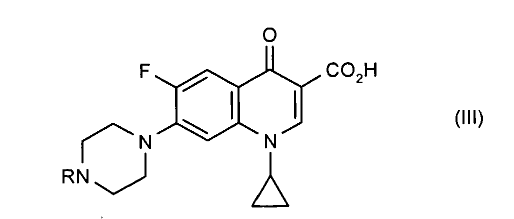 R=H

2. Use of the compound defined in Claim 1 for producing medicaments for the local control of bacterial diseases of the lungs caused by *P. aeruginosa* in humans and animals, where this compound is administered in solid form.

## Revendications

1. Procédé de fabrication d'un médicament pour lutter contre les maladies bactériennes des poumons causées par *P. Aeruginosa* chez les hommes et les animaux par application locale d'une quantité efficace du point de vue antibactérien de bétaïne solide de formule (III) 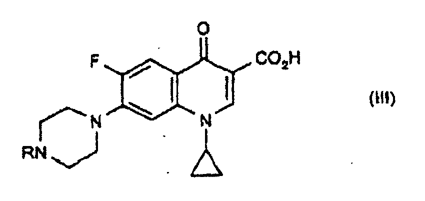 R = H.

2. Utilisation du composé défini dans la revendication 1 pour la fabrication de médicaments pour lutter localement contre les maladies bactériennes des poumons causées par *P. Aeruginosa* chez les hommes et les animaux, ce composé étant appliqué sous forme solide.
